# EUROPÄISCHE PATENTSCHRIFT

(11) **EP 0 930 055 B1**
(45) Veröffentlichungstag und Bekanntmachung des Hinweises auf die Patenterteilung: **31.03.2004**
(21) Anmeldenummer: 98123601.1
(22) Anmeldetag: 10.12.1998
(51) Int. Cl.: A61G 15/14, F16M 11/04

(54) **Medizintechnischer oder dentaltechnischer Arbeitstisch**
Work table for medical or dental application
Table de travail pour techniques médicales ou dentaires

(30) Priorität: 15.01.1998 DE 19801314
(43) Veröffentlichungstag der Anmeldung: 21.07.1999
(73) Patentinhaber: Kaltenbach & Voigt GmbH & Co. KG, 88400 Biberach/Riss (DE)
(72) Erfinder: Buchmann, Siegfried, 88319 Aitrach (DE); Mack, Karl, 88299 Leutkirch (DE)
(74) Vertreter: Schmidt-Evers, Jürgen, Dipl.-Ing.

(56) Entgegenhaltungen:
- WO-A-97/33057
- DE-A- 19 624 506
- FR-A- 1 577 417
- US-A- 4 397 439
- US-A- 4 447 031
- US-A- 4 523 732
- US-A- 4 653 715
- US-A- 5 203 322
- US-A- 5 441 042

## Beschreibung

Die Erfindung bezieht sich auf einen medizintechnischen oder dentaltechnischen Arbeitstisch. Ein solcher Arbeitstisch wird in einem medizintechnischen oder dentaltechnischen Labor oder auch in medizinischen oder dentalmedizinischen Praxen benutzt.

An einen solchen Arbeitstisch besteht die Forderung, verschiedene Arbeiten handhabungsfreundlich und qualitativ durchführen zu können. Hierbei kann es sich z.B. um Bearbeitungsmaßnahmen an einem Werkstück oder Modell oder um Prüfmaßnahmen wie meßtechnische Maßnahmen oder optische Untersuchungen handeln.

Bei einem in der DE 196 24 506 A1 beschriebenen bekannten Arbeitstisch für ein medizinisches oder dentaltechnisches Labor ist die Arbeitsvorrichtung durch eine haubenförmige Schutzvorrichtung mit einer vorderen Schutzscheibe aus durchsichtigem Material gebildet, die den Benutzer bei im Bereich der Schutzvorrichtung durchzuführenden spanabhebenden Bearbeitungen vor Verletzungen durch Bearbeitungsspäne oder auch vor der Verbreitung von bei der Bearbeitung entstehenden Stäuben schützen soll. Um die Sicht auf den Bearbeitungsgegenstand oder den Arbeitsplatz zu verbessern, ist der Arbeitsvorrichtung eine optische Vergrößerungsvorrichtung zugeordnet, die als Anbauteil der Arbeitsvorrichtung ausgebildet und z.B. durch eine Lupe gebildet ist, die an der Frontseite der vorderen Schutzscheibe positionierbar ist.

Der bekannte Arbeitstisch hat sich als brauchbar erwiesen, jedoch ist er in seiner Benutzung eingeschränkt. Es läßt sich zwar die optische Vergrößerungsvorrichtung in einfacher Weise an der als sogenannte Absaughaube ausgebildeten Arbeitsvorrichtung positionieren, jedoch ist die Vergrößerungsvorrichtung nur beim Vorhandensein der Arbeitsvorrichtung zu gebrauchen. Hierdurch ist der Verwendungsbereich des Arbeitstisches eingeschränkt.

Aus der US 5,441,042 ist ferner eine bewegbare medizinische Konsole bekannt, die zur Verwendung in Behandlungsräumen vorgesehen ist. An der Konsole ist ein Gelenkarm befestigt, der an seinem vorderen Ende eine Halterung für ein medizinisches Arbeitsinstrument, insbesondere für eine endoskopische Kamera aufweist. Die Datenübertragung bzw. Stromversorgung von der Kamera zu der Konsole oder in entgegengesetzter Richtung erfolgt dabei über ein Kabel, welches unabhängig von dem Gelenkarm zwischen der Konsole und der Kamera verläuft.

Der Erfindung liegt die Aufgabe zugrunde, einen vorliegenden medizintechnischen oder dentaltechnischen Arbeitstisch so auszugestalten, daß er für einen breiteren Verwendungsbereich einsetzbar ist.

Diese Aufgabe wird durch die Merkmale des Anspruchs 1 gelöst.

Beim erfindungsgemäßen Arbeitstisch sind eine oder mehrere unterschiedliche Arbeitsvorrichtungen vorgesehen, die für eine Inbetriebnahme oder zur Anpassung des Arbeitstisches an spezifische Arbeiten mit dem Verbindungsarm handhabungsfreundlich und schnell verbindbar ist bzw. sind. Hierzu dient eine Schnellkupplung, die eine handhabungsfreundliche und schnelle Montage der Arbeitsvorrichtungen am Verbindungsarm und Demontage vom Verbindungsarm gewährleistet. Bei der erfindungsgemäßen Ausgestaltung kann eine spezifische Arbeitsvorrichtung am Arbeitsplatz des Arbeitstisches positioniert werden, ohne daß die Gefahr einer Behinderung durch eine andere Arbeitsvorrichtung besteht. Dieser Vorteil zeigt sich insbesondere dann, wenn eine Arbeitsvorrichtung in Form einer optischen Vergrößerungsvorrichtung, z.B. ein Mikroskop, vorgesehen ist. Bei der bekannten Ausgestaltung kann lediglich eine solche optische Vergrößerungsvorrichtung verwendet werden, die an der vorhandenen Schutzvorrichtung positionierbar ist, wobei eine von der Schutzvorrichtung unabhängige Positionierung oder Ausrichtung des Mikroskops nicht möglich ist. Dagegen kann bei der erfindungsgemäßen Ausgestaltung die optische Vergrößerungsvorrichtung oder eine spezifische Arbeitsvorrichtung unabhängig am Verbindungsarm gehalten werden und gezielt positioniert und benutzt werden, wodurch die durchzuführenden Arbeiten nicht nur leichter sondern auch mit größerer Präzision und Qualität durchgeführt werden können.

Erfindungsgemäß ist ferner die Schnellkupplungsvorrichtung so ausgebildet, daß vorhandene Medienleitungen wie Zuführungsleitungen für z.B. elektrischen Strom, Druckoder Saugluft oder Licht und Wasser sich durch die Schnellkupplung und somit auch durch die Schnellkupplungsteile erstrecken und durch Medienleitungsabschnitte gebildet sind, die im Bereich der Schnellkupplung ebenfalls durch Schnellkupplungsteile miteinander verbindbar sind. Für die Schnellkupplung eignet sich eine Steckkupplung, die ein Verbinden oder Lösen durch axiales Zusammenstecken oder Auseinanderziehen ermöglichen. Hierzu kann ein Kupplungszapfen dienen, der mit einer ihn aufnehmenden Kupplungsausnehmung verbindbar ist.

Beim Zusammenstecken und beim Auseinanderziehen der Kupplungsteile können zugleich die Medienleitungsabschnitte im Bereich der Steckkupplung miteinander verbunden oder voneinander getrennt werden. Es bedarf somit nur einer Handhabungsmaßnahme zum Verbinden oder Lösen der Arbeitsvorrichtung und zum gleichzeitigen Verbinden oder Trennen der Medienleitungsabschnitte der wenigstens einen Medienleitung.

Bei dem erfindungsgemäßen Arbeitstisch kann ferner der Verbindungsarm nicht nur vertikal verschwenkbar sondern auch in seiner Länge veränderlich und somit handhabungsfreundlich an wahlweise Arbeitsstellungen anpaßbar sein.

Dabei ist es besonders vorteilhaft, das dem Verbindungsarm zugeordnete Verbindungsvorrichtungsteil mit den beiden vorderen Gelenken des Gelenkvierecks zu verbinden. Bei dieser Ausgestaltung verbleibt das Verbindungsvorrichtungsteil unabhängig von der Höheneinstellung des Verbindungsarms bezüglich der Vertikalen immer in der gleichen Position bzw. Neigungsstellung.

Bei einem medizintechnischen oder dentaltechnischen Arbeitstisch sind in vielen Fällen besondere Stellungen der Arbeitsvorrichtung erforderlich. Dies gilt für eine Arbeitsvorrichtung sowohl in Form einer Schutzvorrichtung oder Absaughaube als auch insbesondere in Form einer optischen Vergrößerungsvorrichtung wie ein Mikroskop. Diese Forderungen sind sowohl aus Handhabungsgründen bedingt als auch zur Ausführung bestimmter Arbeiten in besonderen Stellungen und Ausrichtungen vorgegeben, wie es zur visuellen Beobachtung und Ausmessung am zu bearbeitenden Gegenstand erforderlich sein kann.

Bei einer vorteilhafter Weiterbildung der Erfindung ist daher die Arbeitsvorrichtung in einem Gelenk gelagert, das begrenzt allseitige Schwenkbewegungen ermöglicht und vorzugsweise durch aneinander gelagerte Gelenkteile mit sphärischen Lagerflächen im Sinne eines Kugelgelenks gebildet ist. Eine derartig gelagerte Arbeitsvorrichtung ist im Bereich der begrenzten allseitigen Schwenkbarkeit in jede wahlweise Arbeitsstellung verschwenkbar und in dieser Stellung handhabungsfreundlich und bequem zu gebrauchen, so daß die Bedienungsperson ihre Aufmerksamkeit vermehrt auf die Durchführung der Arbeit richten kann, wodurch auch die Qualität der Arbeit verbessert wird.

In weiteren Ansprüchen sind weitere vorteilhafte Weiterbildungsmerkmale der Erfindung enthalten, die eine einfache, kleine und kostengünstige Herstellung gewährleisten, so daß die Arbeitsvorrichtung nicht nur handhabungsfreundlich mit dem Verbindungsarm verbindbar und von diesem wieder lösbar ist sondern auch handhabungsfreundlich zwischen ihrer Arbeitsstellung und einer Bereitschaftstellung und Parkstellung bewegbar ist, wobei sie aufgrund des Gewichtsausgleichs am Verbindungsarm in jeder Höhenstellung verbleibt.

Eine vorteilhafte Ausgestaltung gibt sich im Rahmen der Erfindung dann, wenn mehrere Arbeitsvorrichtungen vorhanden sind, von denen eine eine sogenannte Absaughaube ist, eine weitere durch eine optische Vergrößerungsvorrichtung, z.B. ein Mikroskop, gebildet ist. Im weiteren kann eine Arbeitsvorrichtung auch durch eine Lampe gebildet sein.

Nachfolgend werden die Erfindung und weitere durch sie erzielbare Vorteile anhand von Ausführungsbeispielen und Zeichnungen näher erläutert. Es zeigen
- Fig. 1: einen medizintechnischen oder dentaltechnischen Arbeitstisch in der Vorderansicht;
- Fig. 2: einen sich von hinten nach vorne erstreckenden Verbindungsarm einer auf dem Arbeitstisches angeordneten Tragvorrichtung in der Seitenansicht;
- Fig. 3: den Verbindungsarm im vertikalen Schnitt III-III in Fig. 2;
- Fig. 4: ein Schnellkupplungsteil des Verbindungsarms in der Unteransicht;
- Fig. 5: einen Adaper für den Verbindungsarm im vertikalen Schnitt;
- Fig. 6: den Adapter in der Unteransicht;
- Fig. 7: eine Arbeitsvorrichtung in Form eines Mikroskops in der Seitenansicht;
- Fig. 8: eine Arbeitsvorrichtung in Form einer Absaughaube im vertikalen Schnitt;
- Fig. 9: eine Arbeitsvorrichtung in Form einer Leuchte in der Seitenansicht, teilweise geschnitten;
- Fig. 10: einen vorderen Verbindungsarmabschnitt in der Seitenansicht in abgewandelter Ausgestaltung;
- Fig. 11: eine Tragvorrichtung mit zwei nebeneinander angeordneten Verbindungsarmen in der Draufsicht;
- Fig. 12: einen medizinischen oder dentalmedizinischen Arbeitstisch mit einer Arbeitsvorrichtung in der Seitenansicht in abgewandelter Ausgestaltung;
- Fig. 13: die Arbeitsvorrichtung nach Fig. 12 in perspektivischer Draufsicht von vorne.

Der allgemein mit 1 bezeichnete Arbeitstisch eignet sich insbesondere für dentaltechnische Zwecke, jedoch kann er auch für allgemeine medizintechnische Zwecke benutzt werden.

Der Arbeitstisch 1 besteht aus einer Tischplatte 2 mit einer oberseitigen Tischfläche 2a, zwei seitlich von vorne nach hinten durchgehend angeordneten blockförmigen Tischbeinen 3, in denen frontseitig zugängliche Schubladen oder Schränke angeordnet sind und zwischen denen ein Freiraum 4 für die Beine der an der Bedienungsseite am Arbeitstisch 1 sitzenden Person (nicht dargestellt) vorhanden ist. Desweiteren weist der Arbeitstisch 1 mehrere Arbeitsvorrichtungen auf, von denen eine Arbeitsvorrichtung 5 zwischen einer unter der Tischplatte 2 zurückgeschobenen Nichtsgebrauchsstellung und einer im vorderen Bereich auf der Tischplatte 2 angeordneten Gebrauchsstellung in einer zugehörigen Führung 6 bewegbar ist. Hierbei handelt es sich um eine Absaugvorrichtung 7 mit einer nach hinten oder nach oben austretenden Absaugöffnung 8, in deren Bereich, hier dahinter sich eine Arbeitsstelle auf der Tischfläche 2a oder auf einer Bodenplatte der Absaugvorrichtung 7 befindet.

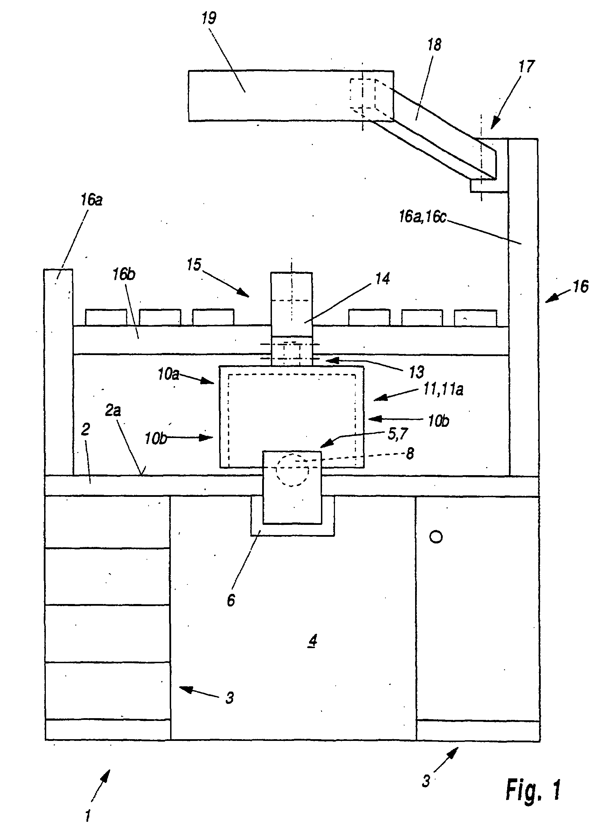

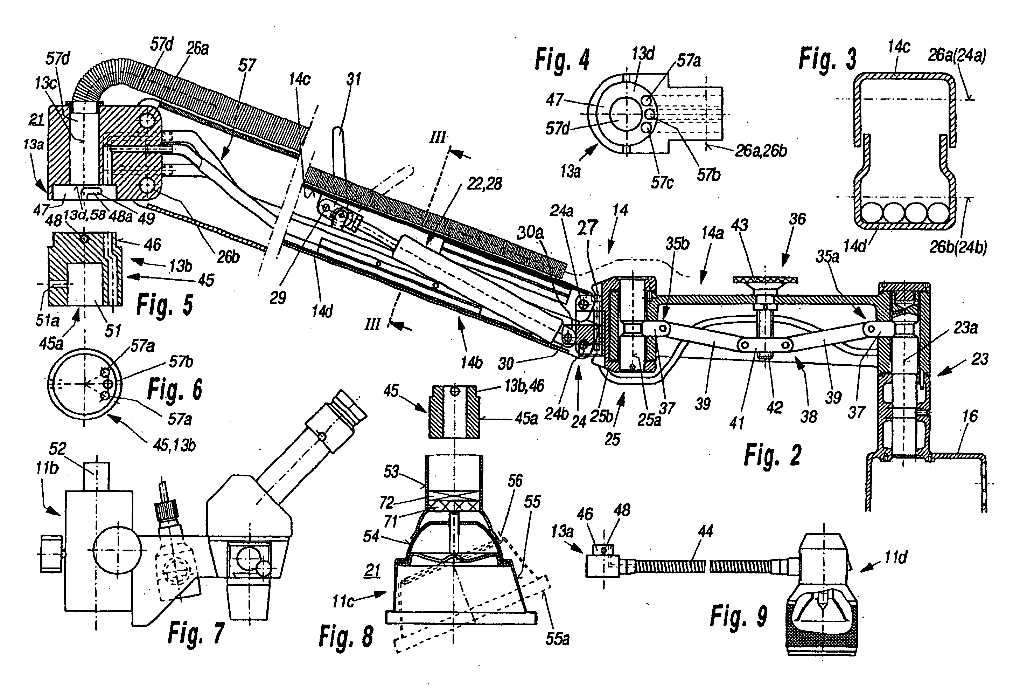

Es sind zwei weitere Arbeitsvorrichtungen 11 vorhanden, nämlich z.B. eine Schutzvorrichtung 11a und eine optische Vergrößerungsvorrichtung 11b (Fig. 7) hier z.B. ein Mikroskop, von denen jeweils eine durch eine Verbindungsvorrichtung in Form einer Schnellkupplung 13 mit einem Verbindungsarm 14 einer Tragvorrichtung 15 lösbar verbindbar ist. In der Fig. 1 ist die Schutzvorrichtung 11a mit dem Verbindungsarm 14 verbunden.

Die Schutzvorrichtung 11a weist eine durchsichtige Frontscheibe 10 auf, die Teil eines sich von ihr nach hinten erstreckenden Schutzgehäuses 10a mit seitlichen Öffnungen 10b für die Hände der Bedienungsperson.

Die Tragvorrichtung 15 weist eine Tragsäule oder ein Traggestell auf, das im hinteren Bereich des Arbeitstisches 1, insbesondere aus diesem, befestigt ist und von diesem nach oben ragt. Bei der vorliegenden Ausgestaltung ist ein viereckiger Tragrahmen 16 mit seitlichen Tragsäulen 16a und einer horizontalen Traverse 16b vorgesehen, von der der Verbindungsarm 14 sich nach vorne erstreckt. An einer nach oben ragenden Verlängerung 16c z.B. der rechten Tragsäule 16a ist durch ein Gelenk 17 mit vertikaler Gelenkachse ein etwa horizontaler Verbindungsarm 18 schwenkbar gelagert, der an seinem nach vorne ragenden freien Ende durch ein weiteres Gelenk mit vertikaler Gelenkachse mit einer Leuchte 19 für den Arbeitstisch 1 horizontal schwenkbar verbunden ist. Auf der Traverse 16b können Ablageplätze, z.B. Vorratsbehälter für Werkzeug oder zu montierende Teile angeordnet sein, die für die Arbeiten auf dem Arbeitstisch 1 benötigt werden.

Die jeweils angekuppelte Arbeitsvorrichtung 11 ist mit Hilfe des Verbindungsarms 14 in ihrer Höhenlage und seitlich und quer zur Arbeitsseite 21 des Arbeitstisches 1 jeweils hin und her bewegbar und vorzugsweise in der jeweiligen Stellung feststellbar. Zur Höheneinstellung kann hierzu eine Gewichtsausgleichsvorrichtung 22 dienen, die noch zu beschreiben ist. Der Beweglichkeit dienen ein Basisgelenk 23 mit vertikaler Gelenkachse 23a und ein Gelenk 24 mit horizontaler Gelenkachse, wobei der Verbindungsarm 14 vorzugsweise als sogenannter Kniehebelgelenkarm mit einem hinteren Verbindungsarmteil 14a und einem vorderen Verbindungsarmteil 14b, die durch ein Kniegelenk 25 mit vertikalter Gelenkachse 25a miteinander verbunden sind. Bei der vorliegenden Ausgestaltung ist das vordere Gelenk 24 an der Vorderseite des Kniegelenks 25 angeordnet. Der Verbindungsarm 14 insgesamt oder hier das vordere Verbindungsarmteil 14b ist durch ein oberes und ein unteres Verbindungsarmteil 14c, 14d gebildet, die im Sinne eines Gelenkvierecks durch ein hinteres und ein vorderes Gelenkpaar, nämlich zwei hintere, vorzugsweise vertikal übereinander angeordnete, Gelenke 24a, 24b und zwei vordere, vorzugsweise vertikal übereinander angeordnete, Gelenke 26a, 26b, miteinander verbunden sind, die vertikal an dem dem vorderen Verbindungsarmteil 14b zugehörigen Gelenkteil 25b des Kniegelenks 25 und an dem Schnellkupplungsteil 13a angeordnet sind.

Zwischen dem unteren Gelenk 24b und dem oberen Verbindungsarmteil 14c, insbesondere in dessen vorderen Längshälfte, ist die Gewichtsausgleichsvorrichtung 22 gelenkig eingeschaltet, z.B. eine Zugfeder (nicht dargestellt) oder eine pneumatische oder hydraulische Feder 28, die in ansich bekannter Weise aus einem Zylinder und einer darin axial verschiebbaren Kolbenstange besteht, die endseitig im hinteren unteren Gelenk 24b und einem am oberen Verbindungsarmteil 14c innen angeordneten Gelenk 29 eingeschaltet ist. Mittels eines auf die Feder 28 einwirkenden Schalthebels 31, der beim vorliegenden Ausführungsbeispiel im freien Endbereich der Kolbenstange schwenkbar angeordnet ist und aus einem das obere Verbindungsarmteil 14c vorzugsweise nach oben durchsetzenden Schlitz herausragt, läßt sich das Gelenkviereck des vorderen Verbindungsarmteils 14b lösen und feststellen bzw. verstarren, wobei das vordere Verbindungsarmteil 14b in der Stellung "lösen" höhenverstellbar ist und in der Stellung "feststellen" in der Höhe fixiert ist. Der als Schwenkhebel ausgebildete Schalthebel 31 kann auf seiner Lagerwelle einen Exzenter aufweisen, der eine in der Kolbenstange axial verschiebbar gelagerte Schaltstange zum Zweck des Lösens und Feststellens verschiebt. Das hintere Ende der Feder 28 ist in einem besonderen Gelenk 30 vertikal schwenkbar gelagert, dessen dem Gelenkteil 25b zugehöriges Gelenkteil 30a durch ein Einstellglied, hier eine Einstellschraube 27, in einer vertikalen Führung vertikal verstellbar und in der jeweiligen Verstellposition feststellbar gelagert ist. Mittels der Einstellschraube 27 läßt sich das vertikal verstellbare Gelenk 30 vertikal justieren, wodurch sich die wirksame Federkraft der Feder 28 verringern und vergrößern und somit an ein Gleichgewicht mit dem wirksamen Gewicht des vorderen Verbindungsarmteils 14b und der damit verbundenen Arbeitsvorrichtung 11 anpassen läßt.

Die übereinander angeordneten vorderen Gelenke 26a, 26b sind an einem Endteil des Verbindungsarms 14 angeordnet, das aufgrund der Anordnung des Gelenkvierecks in jeder Schwenkstellung des Verbindungsarms in seiner vertikalen Ausrichtung unveränderlich ist und das das dem Verbindungsarm 14 zugehörige Schnellkupplungsteil 13a bildet.

Vorzugsweise sind das Basisgelenk 23 und/oder das Kniegelenk 25 mittels einer Feststellvorrichtung 35a, 35b wahlweise manuell feststellbar, die durch eine Betätigungsvorrichtung 36 wahlweise feststellbar oder lösbar ist bzw. sind. Bei der vorliegenden Ausgestaltung ist eine gemeinsame Betätigungsvorrichtung 36 vorgesehen mit jeweils einem radial auf die jeweilige Gelenkachse oder -welle einwirkenden, vorzugsweise keilförmigen Druckbolzen 37, der durch einen Kniehebelmechanismus 38 mit zwei Kniehebelarmen 39 und einem diese Gelenke miteinander verbindenden Kniegelenkstück 41 gegen die zugehörige Gelenkachse drückbar ist. Das Kniegelenkstück 41 ist mittels einer Handschraube 42 quer zu den Kniehebelarmen 39, hier vertikal, verstellbar. Die Handschraube 42 ist drehbar am hinteren Verbindungsarmteil 14a gelagert und durch einen hier am oberen Ende angeordneten Rändelkopf 43 manuell zugänglich und drehbar. Dabei ist das Kniegelenkteil 41 auf einem Gewindeabschnitt der Handschraube 42 beweglich. Die Druckbolzen 37 sind in radialen Löchern der vorhandenen Gelenkbuchsen radial geführt, wobei in den Gelenkachsen vorzugsweise keilförmige Ringnuten angeordnet sind, in die die Druckbolzen 37 radial klemmbar und durch Zurückziehen wieder lösbar sind.

Wie aus Fig. 2 zu entnehmen ist, können dem Arbeitstisch 1 auch andere als wie bereits erwähnte Arbeitsvorrichtungen 11 zugeordnet sein, z.B. eine Absaughaube 11c und eine Leuchte 11d, vorzugsweise mit einem flexiblen Leuchtenarm 44, der sich von einem Kupplungsteil 11b oder Adapter 45 erstreckt (Fig. 9). Die Arbeitsvorrichtungen 11 weisen jeweils ein korrespondierendes Schnellkupplungsteil 13b auf, das mit dem Schnellkupplungsteil 13a handhabungsfreundlich und schnell zu kuppeln ist. Das Kupplungsteil 13b kann direkt an der Arbeitsvorrichtung 11 ausgebildet sein, so daß diese unmittelbar mit dem Schnellkupplungsteil 13a kuppelbar ist, oder das Kupplungsteil 13b kann an einem Adapter 45 ausgebildet sein, der als zusätzliches Bauteil einerseits als Schnellkupplungsteil 13a ausgebildet ist und andererseits spezifische Verbindungsmerkmale 45a aufweist, mit denen er mit der zugehörigen Arbeitsvorrichtung 11 lösbar oder unlösbar verbunden ist.

Die Schnellkupplung 13 ist vorzugsweise eine Steckkupplung mit einer Sicherungsvorrichtung zum Sichern der Schnellkupplungsteile 13a, 13b in der gekuppelten Stellung. Bei der vorliegenden Ausgestaltung weist die Schnellkupplung 13 einen Kupplungszapfen 46 an dem einen Schnellkupplungsteil 13b auf, der in eine Kupplungsausnehmung 47 im anderen Schnellkupplungsteil 13a einsteckbar und axial fixierbar ist, hier mittels eines sogenannten Bajonettverschlusses mit einem oder zwei einander gegenüberliegen angeordneten radialen Verrastungsstiften 48, der bzw. die jeweils hinter eine Hinterschneidung 49 drehbar ist bzw. sind. Bei der vorliegenden Ausgestaltung befindet sich die Kupplungsausnehmung 47 in dem dem Verbindungsarm 14 zugehörigen Schnellkupplungsteil 13a, wobei die Verrastungsstifte 48 radial vom Kupplungszapfen 46 abstehen und jeweils in eine winkelförmige Nut 48a in der Ausnehmungswandung durch Stecken und Drehen einführbar sind. Als Sicherungsvorrichtung kann alternativ eine elastisch nachgiebige und manuell überdrückbare Verrastungsvorrichtung vorgesehen sein.

Zur Befestigung der optischen Vergrößerungsvorrichtung 11b weist der Adapter nach Fig. 6 eine Steckausnehmung 51 auf, in die ein Steckzapfen 52 der Vergrößerungsvorrichtung 11b einsteckbar und durch eine radiale Befestigungsschraube 51a fixierbar ist.

Bei der Absaughaube 11c, die ein hohlzylindrisches Basisteil 53 aufweist, von dem sich kuppelförmig divergent nach unten ein daran schwenkbar gelagertes Trägergehäuse 54 erstreckt, auf dessen kugelabschnittförmig ausgebildeten Kuppelaußenwand eine Haube 55 mit einem Lochrand 56, dessen Querschnittsabmessung kleiner ist als die des Trägergehäuses 54, allseitig schwenkbar gelagert ist, so daß sie mit ihrem unteren Absaugloch 55a in unterschiedliche Stellungen schwenkbar ist. Die Haube 55 kann ebenfalls bezüglich der Arbeitsseite beidseitig Durchgriffsöffnungen 10b für die Hände der Bedienungsperson aufweisen. Ferner kann die Absaughaube 11c oder die Haube 55 wenigstens ein Fenster aufweisen oder vorderseitig aus durchsichtigem Material, insbesondere Kunststoff, bestehen, wodurch die Sicht auf den in der Haube 55 vorhandenen Arbeitsplatz gewährleistet ist.

Um solchen Arbeitsvorrichtungen, die Verbraucher von sogenannten Medien wie elektrischer Strom, Licht, Saugluft und/oder Druckluft sind, das oder die entsprechenden Medien zuzuführen und/oder abzuführen, sind eine oder mehrere Medienleitungen 57 vorgesehen, die sich im oder am Verbindungsarm 14 von hinten nach vorne und durch die Schnellkupplung 13 erstrecken, wobei zueinander gehörige Medienleitungsabschnitte beim Lösen der Schnellkupplung 13 selbsttätig getrennt und beim Schließen der Schnellkupplung 13 selbsttätig verbunden werden. Beim vorliegenden Ausführungsbeispiel sind Medienleitungen z.B. für Kleinspannung und/oder für Licht und/oder für Absaugluft und/oder für Druckluft vorgesehen. Die Medienleitungen 57 sind mit dem am vorderen Ende des Verbindungsarms 14 angeordneten Schnellkupplungsteil 13a verbunden, und sie durchsetzen beide Schnellkupplungsteile 13a, 13b, wobei sie die Teilungsfuge 58 zwischen den Schnellkupplungsteilen 13a, 13b durchsetzen, bei der vorliegenden Ausgestaltung im Bereich eines sich bezüglich der Kupplungsachse 13c radial erstreckenden Teilungsfugenabschnitts 13d. Zur Abdichtung der Medienleitungsenden an der Teilungsfuge 58 können die Medienleitungsöffnungen umgebende Ringdichtungen vorgesehen sein, die in das eine Kupplungsteil eingelassen sind und mit der gegenüberliegenden Wand des anderen Kupplungsteils dichtend zusammenwirken. Wie insbesondere aus Fig. 4 und 6 zu entnehmen ist, liegen die Öffnungen der Medienleitungen 57 in Umfangsrichtung nebeneinander, wobei sie in gleichen oder in unterschiedlichen radialen Abständen von der Kupplungsachse 13c angeordnet sein können. Bei der vorliegenden Ausgestaltung sind mehrere im gleichen radialen Abstand von der Kupplungsachse 13c angeordnete Medienleitungen 57a, 57b, 57c und eine koaxial, d.h. mit dem Radius 0 angeordnete Medienleitung 57d vorgesehen. Die Medienleitungen 57 können an der Rückseite des Schnellkupplungsteils 13a angeschlossen sein und das Schnellkupplungsteil 13a in Winkelkanälen durchsetzen, oder an der Oberseite angeschlossen sein, wobei das entfernbare Schnellkupplungsteil 13b von unten mit dem am Verbindungsarm 14 angeordneten Schnellkupplungsteil 13a verbindbar ist. Beim Vorhandenseins eines Adapters 45 durchsetzen die Medienleitungen 57 diesen ebenfalls, und sie erstrecken sich in der zugehörigen Arbeitsvorrichtung 11 weiter. Nicht benötigte Medienleitungen sind im jeweiligen Schnellkupplungsteil 13b oder Adapter 45 nicht vorhanden, wodurch das Schnellkupplungsteil 13b oder der jeweilige Adapter 45 einen Verschluß für diese Medienleitung 57 bildet.

Bei der vorliegenden Ausgestaltung ist die koaxial im Schnellkupplungsteil 13a verlaufende Medienleitung 57d die im Querschnitt größte Medienleitung, bei der es sich um eine Absaugleitung handelt.

Beim in Fig. 2 dargestellten Verbindungsarm 14 verläuft die Absaugleitung 57d an der Oberseite des Verbindungsarms 14 bis in dessen hinteren Bereich, wobei es sich um eine flexible Medienleitung, z.B. einen Schlauch, der die Bewegungen des Verbindungsarms 14 mitmachen kann.

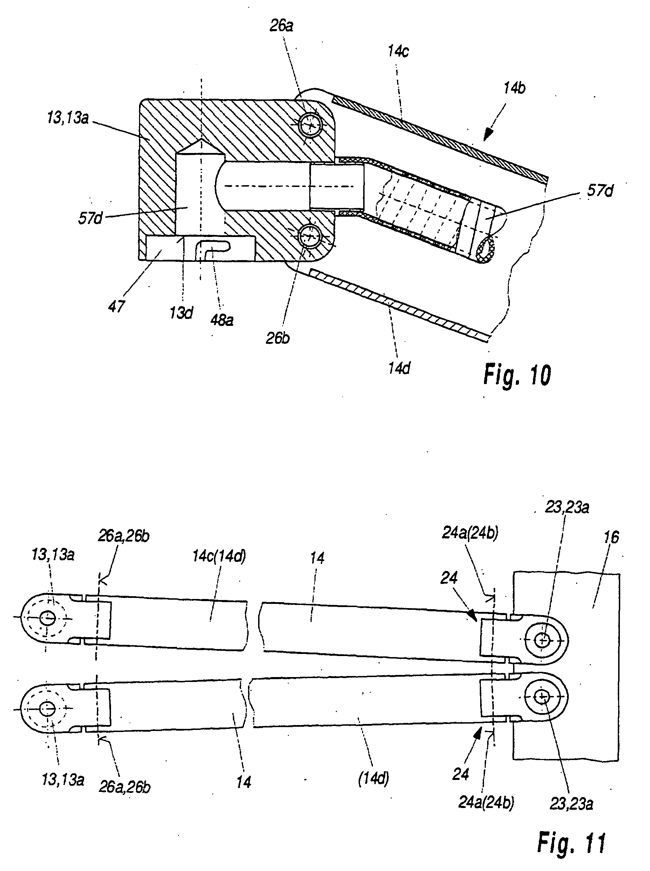

Beim Ausführungsbeispiel nach Fig. 10 verläuft die Absaugleitung 57d mit oder ohne Schlauch zumindest längs durch den vorderen Verbindungsarmteil 14b und als Winkelkanal im Schnellkupplungsteil 13a in die Kupplungsausnehmung 47, wobei andere Medienleitungen nicht vorgesehen sein können und diese Ausgestaltung speziell für eine Absaughaube 11c eingerichtet ist.

Wie insbesondere aus Fig. 3 zu entnehmen ist, bestehen das obere und untere Verbindungsarmteil 14c, 14d aus zwei Verbindungsarmschienen U-förmiger Querschnittsform, deren Schenkel aufeinanderzu gerichtet sind und einander übergreifen, wobei vorzugsweise die Schenkel des oberen Verbindungsarmteils 14c die Schenkel des unteren Verbindungsarmteils 14d mit geringem Bewegungsspiel übergreifen. Dieser Übergriff ist bereits dann vorhanden, wenn die Verbindungsarmteile 14c, 14d ihre Gelenkachsen ihren größten Abstand voneinander aufweisen. Wie aus Fig. 3 ebenfalls zu entnehmen ist, können die Medienleitungen 57 in vorteilhafter Weise insbesondere im Bodenbereich des unteren Verbindungsarmteils 14d angeordnet werden. Im Bereich des Kniegelenks 25 verlaufen die Medienleitungen 57 in einer das Kniegelenk 25 umlaufenden Position, z.B. seitlich oder unterseitig, wobei sie in der Nähe des Kniegelenks 25 aus dem vorderen Verbindungsarmteil 14b austreten.

Im Rahmen der Erfindung können zwei Verbindungsarme 14 nebeneinander in einem gemeinsamen Basisgelenk 23 oder in zwei nebeneinander angeordneten Basisgelenken 23 (Fig. 11) vorgesehen sein, wobei es sich um Verbindungsarme 14 mit vorbeschriebenen Gelenkvierecken und mit oder ohne Kniegelenk 25 handeln kann. Diese Ausgestaltung ermöglicht es z.B., einen oder beide Verbindungsarme jeweils als Träger für eine Leuchte und/oder für eine andere Arbeitsvorrichtung, z.B. eine Schutzvorrichtung 11a und/oder optische Vergrößerungsvorrichtung 11b und/oder Absaughaube 11c zu benutzen.

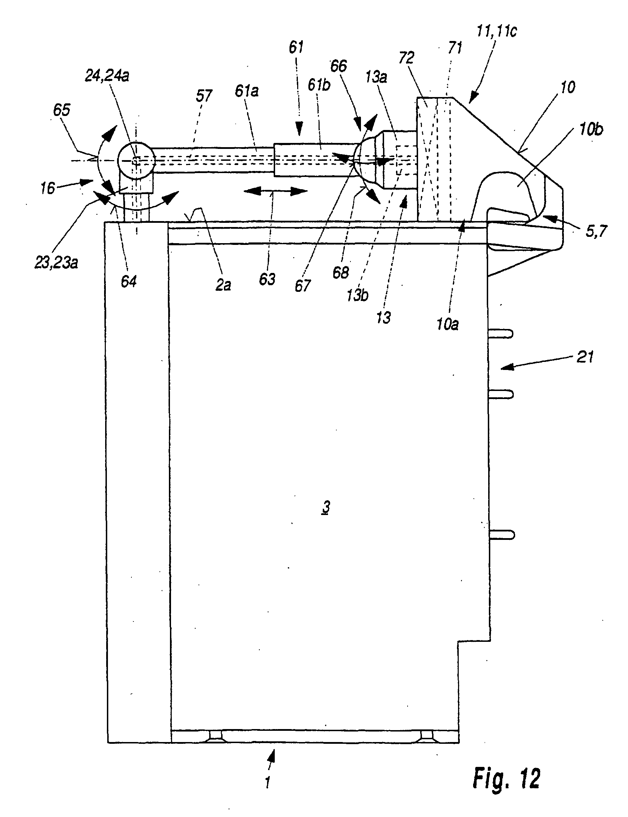

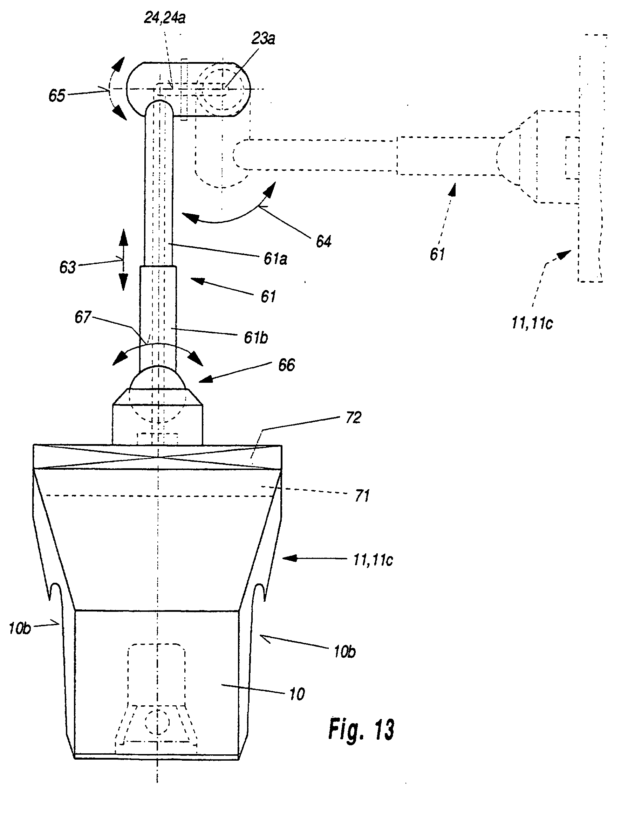

Das Ausführungsbeispiel nach Fig. 12 und 13 unterscheidet sich von dem vorbeschriebenen Ausführungsbeispiel durch einen Verbindungsarm 61 in abgewandelter Ausgestaltung und gegebenenfalls auch eine niedriger ausgebildete oder fehlende Tragvorrichtung 16. Letzteres kann z.B. dadurch verwirklicht sein, daß das Basisgelenk 23 direkt auf der Tischplatte 2 oder auf einem kleinen Sockel angeordnet ist. Der Verbindungsarm 61 ist ein aus wenigstens zwei Verbindungsarmteilen 61a, 61b bestehender Teleskoparm, der wahlweise manuell teleskopierbar ist und in seiner jeweiligen Teleskopstellung vorzugsweise durch eine nicht dargestellte Feststellvorrichtung feststellbar sein kann. Dieser Freiheitsgrad ist durch den Doppelpfeil 63 verdeutlicht. Weitere Freiheitsgrade sind durch die Doppelpfeile 64, 65 verdeutlicht, nämlich die freie Schwenkbarkeit um die vertikale Gelenkachse 23a und um die horizontale Schwenkachse 24a des Gelenks 24, das zwischen dem hinteren Teleskoparm 61a und dem oberen Gelenkteil des Gelenks 23 gebildet sein kann.

Im vorderen Bereich des Verbindungsarms 61 ist ein besonderes Gelenk 66 in Form eines allseitig begrenzt schwenkbaren Raum- oder Kugelgelenks 66 vorgesehen, das eine entsprechende Schwenkbarkeit der Arbeitsvorrichtung 11 ermöglicht, die beim dargestellten Ausführungsbeispiel ebenfalls durch eine optische Vergrößerungsvorrichtung oder eine Absaughaube 11c gebildet sein kann. Das Raumgelenk 66 kann direkt zwischen der Arbeitsvorrichtung 11 und dem Verbindungsarm 61 angeordnet sein. Bei der vorliegenden Ausgestaltung ist zwischen der Arbeitsvorrichtung 11 und dem Verbindungsarm 61 die Schnellkupplung 13 angeordnet, wobei das Raumgelenk 66 zwischen dem dem Verbindungsarm 61 zugehörigen Schnellkupplungsteil 13a und dem vorderen Verbindungsarmteil 61b angeordnet ist. Es ist im Rahmen der Erfindung jedoch auch möglich, das Raumgelenk 66 zwischen der Arbeitsvorrichtung 11 und dem ihr zugeordneten Schnellkupplungsteil 13b oder zwischen zwei anderen Arbeitsvorrichtungsteilen anzuordnen. Die allseitig begrenzte Schwenkbarkeit des Raumgelenks 66 ist durch zwei Doppelpfeile 67, 68 verdeutlicht.

Auch bei diesem Verbindungsarm 61 ist bzw. sind eine oder mehrere Medienleitungen 57 bzw. 57a bis 57d außen am Verbindungsarm 61 oder längs durch den Verbindungsarm 61 verlaufend angeordnet, letzteres ist durch gestrichelte Linien andeutungsweise dargestellt. Bei einem Verlauf der wenigstens einen Mediumleitung 57 am Umfang des Verbindungsarms 61 kann diese am vorderen Verbindungsarmteil 61b gehalten sein, wobei der hintere Abschnitt der Mediumleitung sich frei erstrecken und beim Teleskopieren des vorderen Verbindungsarmteils 61b frei folgen kann. Fig. 13 zeigt den Verbindungsarm 61 mit der Arbeitsvorrichtung 11 in einer seitlich nach hinten geschwenkten Bereitschaftsoder Parkstellung. Sowohl in dieser Stellung als auch in seiner Arbeitsstellung kann die Arbeitsvorrichtung 11 auf der Tischplatte 2 aufliegen. Sofern eine Feststellbarkeit der Arbeitsvorrichtung 11 bezüglich des Verbindungsarms 61 oder des Verbindungsarms 61 im Gelenk 24 gewünscht ist bzw. sind, ist dem jeweiligen Gelenk 26 bzw. 24 eine Feststellvorrichtung zuzuordnen, die ein Feststellen des Gelenks in der eingestellten Stellung ermöglicht. Dies kann z.B. durch eine Schwergängigkeit des betreffenden Gelenks erreicht werden, die so groß ist, daß eine manuelle Verstellung gewährleistet ist, jedoch eine unbeabsichtigte Verstellung verhindert ist. Letzteres gilt auch für die Teleskopierbarkeit insbesondere dann, wenn entweder in nach oben geschwenkten Schrägstellungen des Verbindungsarms 61 oder bei einem höheren Sockel 62 in nach unten geschwenkten Schrägstellungen ein selbsttätiges Ein- oder Ausschieben des Verbindungsarms 61 verhindert sein soll. Auch in diesem Fall kann die Feststellvorrichtung durch eine Schwergängigkeit zwischen den Teleskopteilen gebildet sein.

Bei den vorbeschriebenen Ausführungsbeispielen ist in den Fällen, in denen die Arbeitsvorrichtung 11 durch eine Absaughaube 11c gebildet ist, der Arbeitstisch 1 mit einer Absaugleitung 57d beschrieben, die sich von der Absaughaube 11c zu einer nicht dargestellten Saugvorrichtung im Bereich des Arbeitstisches oder zu einer zentralen Absaugvorrichtung erstreckt, an die die Absaugleitungen mehrerer Arbeitstische angeschlossen sein können. In diesen Fällen ist der Einzel-Absaugvorrichtung oder der zentralen Absaugvorrichtung jeweils eine Filtervorrichtung mit einem Filter zum Filtern von abgesaugten Partikeln oder Verunreinigungen zugeordnet.

Im Rahmen der Erfindung ist es jedoch auch möglich, ein Filter 71 oder ein Filter 71 und eine Saugvorrichtung 72 an der Absaughaube 11c anzuordnen, insbesondere in deren hinterem oder oberem Bereich, wie es die Fig. 8 und 12 sowie 13 zeigen. Bei der Ausgestaltung nach Fig. 8 ist in Absaugrichtung zunächst der Filter 71 und dahinterliegend die Saugvorrichtung 72, z.B. ein Ventilator, im Basisteil 53 dieser Absaughaube 11c angeordnet, das sich in Form eines Rohrstutzens nach oben erstreckt. Bei der Ausgestaltung nach Fig. 12 bzw. 13 sind der Filter 71 und die Saugvorrichtung 72 entsprechend im hinteren Bereich der Arbeitsvorrichtung 11c angeordnet. Bei dieser Ausgestaltung erfüllt die zugehörige Medienleitung nicht die Funktion einer Absaugleitung sondern einer Abführungsleitung für die im Bereich der Absaughaube eingesaugte Abluft.

Bei den vorbeschriebenen Ausführungsbeispielen, bei denen der Verbindungsarm 14, 61 in einer mit der zugehörigen Arbeitsvorrichtung 11 von der Tischfläche 2a abgehobenen Position positionierbar oder feststellbar ist, bildet der Verbindungsarm 14, 61 einen Tragarm für die Arbeitsvorrichtung 11. Wenn eine solche Höhenpositionierung bzw. - feststellung nicht vorgesehen ist und die Arbeitsvorrichtung 11 auf der Tischfläche 2a aufliegt, s. z.B. Absaughaube 11c in Fig. 12, dann übt der Verbindungsarm die Funktion einer Verbindung oder Führung für die Arbeitsvorrichtung 11 aus.

## Patentansprüche

1. Medizintechnischer oder dentaltechnischer Arbeitstisch (1) mit wenigstens einer darauf angeordneten Arbeitsvorrichtung (11), die durch eine Verbindungsvorrichtung mit einem Verbindungsarm (14, 61) lösbar verbunden und horizontal sowie auf- und abwärts zwischen einer Bereitschafts- oder Parkstellung und einer Arbeitsstellung bewegbar ist,
wobei die Verbindungsvorrichtung durch eine Schnellkupplung (13) mit einem am Verbindungsarm (14, 61) angeordneten Schnellkupplungsteil (13a) und einem an der Arbeitsvorrichtung (11) angeordneten Schnellkupplungsteil (13b) gebildet ist
**dadurch gekennzeichnet,**
**daß** eine oder mehrere zusätzliche Arbeitsvorrichtungen (11) unterschiedlicher Art jeweils mit einem Schnellkupplungsteil (13b) vorgesehen ist bzw. sind, das mit dem Schnellkupplungsteil (13a) am Verbindungsarm (14) kuppelbar ist,
wobei am Verbindungsarm (14, 61) eine oder mehrere längs verlaufende Medienleitungen (57) vorgesehen ist bzw. sind, die sich wenigstens teilweise durch den Verbindungsarm (14, 61) und durch die Schnellkupplungsteile (13a, 13b) erstreckt bzw. erstrecken und eine zwischen den Schnellkupplungsteilen (13a, 13b) vorhandene Teilungsfuge (58) quer durchsetzt bzw. durchsetzen.

2. Medizintechnischer oder dentaltechnischer Arbeitstisch nach Anspruch 1,
**dadurch gekennzeichnet,**
**daß** die Schnellkupplung (13) eine Steckkupplung mit einer Kupplungsausnehmung (47) und einen darin einsteckbaren Kupplungszapfen (46) und eine Sicherungsvorrichtung zum axialen Sichern der Kupplungsteile in der gekuppelten Stellung gebildet ist.

3. Medizintechnischer oder dentaltechnischer Arbeitstisch nach Anspruch 2,
**dadurch gekennzeichnet,**
**daß** die axiale Sicherungsvorrichtung durch einen Bajonettverschluß oder eine elastisch nachgiebige, manuell überdrückbare Verrastungsvorrichtung gebildet ist.

4. Medizintechnischer oder dentaltechnischer Arbeitstisch nach einem der vorherigen Ansprüche,
**dadurch gekennzeichnet,**
**daß** die Medienleitung oder Medienleitungen (57) die Grundfläche (13d) der Kupplungsausnehmung (47) und die Stirnfläche des Kupplungszapfens (46) durchsetzen.

5. Medizintechnischer oder dentaltechnischer Arbeitstisch nach einem der vorherigen Ansprüche,
**dadurch gekennzeichnet,**
**daß** das an der Arbeitsvorrichtung (11) angeordnete Schnellkupplungsteil (13b) an einem Adapter (45) ausgebildet ist, der mit der Arbeitsvorrichtung (11) verbunden ist.

6. Medizintechnischer oder dentaltechnischer Arbeitstisch nach einem der vorherigen Ansprüche,
**dadurch gekennzeichnet,**
**daß** der Verbindungsarm (14) horizontal und vertikal schwenkbar gelagert ist und vorzugsweise durch eine Feststellvorrichtung oder eine Gewichtsausgleichsvorrichtung in jeder Höhenlage positionierbar ist oder positioniert ist.

7. Medizintechnischer oder dentaltechnischer Arbeitstisch nach Anspruch 6,
**dadurch gekennzeichnet,**
**daß** die Ausgleichskraft der Gewichtsausgleichsvorrichtung vergrößerbar und verringerbar ist.

8. Medizintechnischer oder dentaltechnischer Arbeitstisch nach Anspruch 6 oder 7,
**dadurch gekennzeichnet,**
**daß** die Feststellvorrichtung oder Gewichtsausgleichsvorrichtung durch ein Schaltelement in und außer Funktion setzbar ist.

9. Medizintechnischer oder dentaltechnischer Arbeitstisch nach einem der vorherigen Ansprüche,
**dadurch gekennzeichnet,**
**daß** der Verbindungsarm (14) oder das vordere Verbindungsarmteil (14b) aus einem oberen und einem unteren Verbindungsarmteil (14c, 14d) besteht, die durch endseitig übereinander angeordnete vier Gelenke (24a, 24b, 26a, 26b) im Sinne eines Gelenkvierecks miteinander verbunden sind.

10. Medizintechnischer oder dentaltechnischer Arbeitstisch nach einem der vorherigen Ansprüche,
**dadurch gekennzeichnet,**
**daß** die Medienleitung oder die Medienleitungen (57) sich wenigstens teilsweise längs durch den Verbindungsarm (14) oder das vordere Verbindungsarmteil (14b) erstrecken.

11. Medizintechnischer oder dentaltechnischer Arbeitstisch nach einem der vorherigen Ansprüche
**dadurch gekennzeichnet,**
**daß** der Verbindungsarm (14) aus einem hinteren und einem vorderen Verbindungsarmteil (14a, 14b) besteht, die durch ein Mittelgelenk (25) mit im wesentlichen vertikal verlaufender Gelenkachse (25a) miteinander verbunden sind.

12. Medizintechnischer oder dentaltechnischer Arbeitstisch (1) nach Anspruch 11
**dadurch gekennzeichnet**
**daß** die Verbindungsarmteile (14a, 14b) durch das Mittelgelenk (25) schwenkbar miteinander verbunden sind, und das vordere Verbindungsarmteil (14b) aus einem oberen und einem unteren Verbindungsarmteil (14c, 14d) besteht, die an ihren hinteren und vorderen Enden jeweils durch zwei übereinander angeordnete Gelenke (24a, 24b, 26a, 26b) im Sinne eines Gelenkvierecks miteinander und mit dem Mittelgelenk (25) verbunden sind.

13. Medizintechnischer oder dentaltechnischer Arbeitstisch nach Anspruch 12,
**dadurch gekennzeichnet,**
**daß** das obere und das untere Verbindungsarmteil (14c, 14d) aus U-Schienen bestehen, deren Schenkel aufeinander zugerichtet sind, wobei die Schenkel vorzugsweise des oberen Verbindungsarmteils (14c) die Schenkel des anderen Verbindungsarmteils (14d) übergreifen.

14. Medizintechnischer oder dentaltechnischer Arbeitstisch nach einem der Ansprüche 11 bis 13,
**dadurch gekennzeichnet.**
**daß** ein am hinteren Ende des Verbindungsarms (14) angeordnetes Basisgelenk (23) und/oder das Mittelgelenk (25) wahlweise durch eine jeweils zugehörige oder gemeinsame Feststellvorrichtung (36) feststellbar ist bzw. sind.

15. Medizintechnischer oder dentaltechnischer Arbeitstisch (1) nach einem der vorherigen Ansprüche
**dadurch gekennzeichnet,**
**daß** die Arbeitsvorrichtung (11) durch ein Gelenk (66) begrenzt räumlich schwenkbar gelagert ist.

16. Medizintechnischer oder dentaltechnischer Arbeitstisch nach Anspruch 15,
**dadurch gekennzeichnet,**
**daß** das Gelenk (66) zwischen der Arbeitsvorrichtung (11) und dem ihr zugehörigen Verbindungsvorrichtungsteil oder zwischen zwei Teilen der Arbeitsvorrichtung (11) oder zwischen dem Verbindungsarm (14) und dem ihm zugehörigen Verbindungsvorrichtungsteil angeordnet ist.

17. Medizintechnischer oder dentaltechnischer Arbeitstisch nach einem der vorherigen Ansprüche,
**dadurch gekennzeichnet,**
**daß** der Verbindungsarm (14) in Form einer Teleskopstange ausgebildet ist, manuell teleskopierbar ist und vorzugsweise in der jeweiligen Teleskopstellung durch eine Feststellvorrichtung feststellbar ist.

## Claims

1. Medical technical or dental technical work table (1) having at least
a work device (11) arranged thereon, which work device is releasably connected, by way of a connection device, with a connection arm (14, 61) and is moveable horizontally and up and down between a standby or parked position and a work position,
the connection device being formed by means of a quick coupling (13) having a quick coupling part (13a) arranged on the connection arm (14, 61) and a quick coupling part (13b) arranged on the work device (11),
**characterized in that**,
there is or are provided one or more additional work devices (11) of different kinds, in each case with a quick-coupling part (13b) which can be coupled with the quick-coupling part (13a) on the connection arm (14),
there being provided on the connection arm (14, 61) one or more longitudinally running media lines (57) which extends or extend at least in part through the connection arm (14, 61) and through the quick coupling parts (13a, 13b) and passes or pass transversely through a separating joint (58) present between the quick coupling parts (13a, 13b).

2. Medical technical or dental technical work table according to claim 1,
**characterized in that**
the quick coupling (13) is a plug-in coupling having a coupling recess (47) and a coupling pin (46) insertable therein, and a securing device for axially securing the coupling parts in the coupled position.

3. Medical technical or dental technical work table according to claim 2,
**characterized in that**,
the axial securing device is formed by means of a bayonet fastening or an elastically yieldable latching device which can be manually overcome.

4. Medical technical or dental technical work table according to any preceding claim,
**characterized in that**,
the media line or media lines (57) pass through the base surface (13d) of the coupling recess (47) and the end face of the coupling pin (46).

5. Medical technical or dental technical work table according to any preceding claim,
**characterized in that**,
there is formed on the quick coupling part (13b) arranged on the work device (11) an adapter (45) which is connected with the work device (11).

6. Medical technical or dental technical work table according to any preceding claim,
**characterized in that**,
the connection arm (14) is mounted horizontally and vertically pivotably and preferably is or can be positioned in any height disposition by means of a fixing device or a weight balancing device.

7. Medical technical or dental technical work table according to claim 6,
**characterized in that**,
the balancing force of the weight balancing device can be increased and decreased.

8. Medical technical or dental technical work table according to claim 6 or 7,
**characterized in that**,
the fixing device or weight balancing device can be put into or out of function by means of a switch element.

9. Medical technical or dental technical work table according to any preceding claim,
**characterized in that**,
the connection arm (14), or the forward connection arm part (14b), is of an upper and a lower connection arm part (14c, 14d) which are connected with one another by means of four joints (24a, 24b, 26a, 26b) arranged at the ends above one another, in the manner of a four bar linkage.

10. Medical technical or dental technical work table according to any preceding claim,
**characterized in that**,
the media line or the media lines (57) extend at least in part longitudinally through the connection arm (14) or the forward connection arm part (14b).

11. Medical technical or dental technical work table according to any preceding claim,
**characterized in that**,
the connection arm (14) is of a rearward and a forward connection arm part (14a, 14b) which are connected with one another by means of a middle joint (25) having a substantially vertically running joint axis (25).

12. Medical technical or dental technical work table (1) according to claim 11,
**characterized in that**,
the connection arm parts (14a, 14b) are pivotably connected with one another by means of the middle joint (25), and the forward connection arm part (14b) is of an upper and a lower connection arm part (14c, 14d) which are connected at their rearward and forward ends with the middle joint (25) in each case by means of two joints (24a, 24b, 26a, 26b) arranged above one another in the manner of a four bar linkage.

13. Medical technical or dental technical work table according to claim 12,
**characterized in that**,
the upper and the lower connection arm parts (14c, 14d) are of U-rails, the limbs of which are directed towards one another, whereby the limbs - preferably of the upper connection arm part,(14c) - engage over the limbs of the other connection arm part (14d).

14. Medical technical or dental technical work table according to any of claims 11 to 13,
**characterized in that**,
a base joint arranged on the rearward end of the connection arm (14) and/or the middle joint (25) is or are selectively fixable by means of a respectively associated or common fixing device (36).

15. Medical-technical or dental technical work table (1) according to any preceding claim,
**characterized in that**,
the work device (11) is mounted by means of a joint (66) to be spatially restrictedly pivotable.

16. Medical technical or dental technical work table according to claim 15,
**characterized in that**,
the joint (66) is arranged between the work device (11) and the connection device part associated therewith, or between two parts of the work device (11) or between the connection arm (14) and the connection device part associated therewith.

17. Medical technical or dental technical work table according to any preceding claim,
**characterized in that**,
the connection arm (14) is formed in the manner of a telescopic rod, can be telescoped manually and preferably can be fixed in the respective telescoped position by means of a fixing device.

## Revendications

1. Table de travail (1) pour techniques médicales ou dentaires comprenant au moins un dispositif de travail (11) disposé dessus, qui est relié de façon amovible par un dispositif de liaison à un arbre de liaison (14, 61) et peut être déplacé horizontalement ainsi que vers le haut et vers le bas entre une position en attente ou de parcage et une position de travail, le dispositif de liaison étant formé par une attache rapide (13) avec une partie d'attache rapide (13a) disposée sur le bras de liaison (14, 61) et une partie d'attache rapide (13b) disposée sur le dispositif de travail (11),
**caractérisée en ce que**
il est prévu un ou plusieurs dispositifs de travail (11) supplémentaires de type différent pourvu chacun d'une partie d'attache rapide (13b), qui peut être couplée avec la partie d'attache rapide (13a) sur le bras de liaison (14),
une ou plusieurs conduites pour fluides (57) disposée(s) dans le sens de la longueur étant prévue(s) sur le bras de liaison (14, 61), qui s'étend ou s'étendent au moins partiellement à travers le bras de liaison (14, 61) et à travers les parties d'attache rapide (13a, 13b) et traverse(nt) transversalement un joint de séparation (58) présent entre les parties d'attache rapide (13a, 13b).

2. Table de travail pour techniques médicales ou dentaires selon la revendication 1,
**caractérisée en ce que**
l'attache rapide (13) est formée par une attache emboîtable avec un évidement d'attache (47) et un tenon d'attache (46) emboîtable à l'intérieur de celui-ci et un dispositif de blocage pour le blocage axial des parties d'attache dans la position attachée.

3. Table de travail pour techniques médicales ou dentaires selon la revendication 2,
**caractérisée en ce que**
le dispositif de blocage axial est formé par une fermeture à baïonnette ou un dispositif d'encliquetage élastique et souple et pouvant être forcé manuellement.

4. Table de travail pour techniques médicales ou dentaires selon l'une quelconque des revendications précédentes,
**caractérisée en ce que**
la conduite pour fluides ou les conduites pour fluides (57) traversent la surface de base (13d) de l'évidement d'attache (47) et la surface frontale du tenon d'attache (46).

5. Table de travail pour techniques médicales ou dentaires selon l'une quelconque des revendications précédentes,
**caractérisée en ce que**
la partie d'attache rapide (13b) disposée sur le dispositif de travail (11) est conçue sur un adaptateur (45) qui est relié au dispositif de travail (11).

6. Table de travail pour techniques médicales ou dentaires selon l'une quelconque des revendications précédentes,
**caractérisée en ce que**
le bras de liaison (14) est logé de façon basculante horizontalement et verticalement et peut être positionné ou est positionné de préférence par un dispositif de fixation ou un dispositif d'équilibrage de poids dans toute position en hauteur.

7. Table de travail pour techniques médicales ou dentaires selon la revendication 6,
**caractérisée en ce que**
la force d'équilibrage du dispositif d'équilibrage de poids peut être augmentée ou diminuée.

8. Table de travail pour techniques médicales ou dentaires selon la revendication 6 ou 7,
**caractérisée en ce que**
le dispositif de fixation ou le dispositif d'équilibrage de poids peut être mis en fonction ou hors fonction par un élément de commutation.

9. Table de travail pour techniques médicales ou dentaires selon l'une quelconque des revendications précédentes,
**caractérisée en ce que**
le bras de liaison (14) ou la partie avant du bras de liaison (14b) comprend une partie supérieure de bras de liaison et une partie inférieure de bras de liaison (14c, 14d), qui sont reliées entre elles par quatre articulations (24a, 24b, 26a, 26b) disposées l'une au-dessus de l'autre, du côté extrémité, dans l'optique d'un quadrilatère articulé.

10. Table de travail pour techniques médicales ou dentaires selon l'une quelconque des revendications précédentes,
**caractérisée en ce que**
la conduite pour fluides ou les conduites pour fluides (57) s'étendent au moins en partie dans le sens de la longueur à travers le bras de liaison (14) ou la partie avant du bras de liaison (14b).

11. Table de travail pour technique médicale et dentaire selon l'une quelconque des revendications précédentes,
**caractérisée en ce que**
le bras de liaison (14) comprend une partie de bras de liaison arrière et une partie de bras de liaison avant (14a, 14b), qui sont reliées entre elles par une articulation centrale (25) avec un axe d'articulation (25a) agencé sensiblement verticalement.

12. Table de travail (1) pour techniques médicales et dentaires selon la revendication 11,
**caractérisée en ce que**
les parties de bras de liaison (14a, 14b) sont reliées entre elles de façon pivotante par l'articulation centrale (25), et la partie avant du bras de liaison (14b) comprend une partie supérieure de bras de liaison et une partie inférieure de bras de liaison (14c, 14d), qui sont reliées entre elles et à l'articulation centrale (25) sur leurs extrémités arrière et avant respectivement par deux articulations (24a, 24b, 26a, 26b) disposées l'une au-dessus de l'autre dans l'optique d'un quadrilatère articulé.

13. Table de travail pour techniques médicales ou dentaires selon la revendication 12,
**caractérisée en ce que**
la partie avant du bras de liaison et la partie inférieure de bras de liaison (14c, 14d) consistent en des rails en U dont les branches sont orientées l'une vers l'autre, les branches de préférence de la partie supérieure de bras de liaison (14c) recouvrant les branches de l'autre partie de bras de liaison (14d).

14. Table de travail pour techniques médicales ou dentaires selon l'une quelconque des revendications 11 à 13,
**caractérisée en ce que**
une articulation de base (23) disposée sur l'extrémité arrière du bras de liaison (14) et/ou l'articulation centrale peut être ou peuvent être fixées au choix par un dispositif de blocage respectif ou commun.

15. Table de travail pour techniques médicales ou dentaires selon l'une quelconque des revendications précédentes,
**caractérisée en ce que**
le dispositif de travail (11) est logé de façon à pouvoir basculer dans l'espace de manière limitée par une articulation (66).

16. Table de travail pour techniques médicales ou dentaires selon la revendication 15,
**caractérisée en ce que**
l'articulation (66) est disposée entre le dispositif de travail (11) et la partie du dispositif de liaison qui lui correspond ou entre deux parties du dispositif de travail (11) ou entre le bras de liaison (14) et la partie du dispositif de liaison qui lui correspond.

17. Table de travail pour techniques médicales ou dentaires selon l'une quelconque des revendications précédentes
**caractérisée en ce que**
le bras de liaison (14) est conçu sous la forme d'une tige télescopique, peut être entré et sorti manuellement et peut être fixé de préférence dans chaque position sortie par un dispositif de fixation.
